# EUROPÄISCHE PATENTSCHRIFT

(11) **EP 0 313 935 B1**
(45) Veröffentlichungstag und Bekanntmachung des Hinweises auf die Patenterteilung: **27.07.1994**
(21) Anmeldenummer: 88117090.6
(22) Anmeldetag: 14.10.1988
(51) Int. Cl.: C07D 513/04, A61K 31/54

(54) **Enolether von 6-Chlor-4-hydroxy-2-methyl-N-(2-pyridyl)-2H-thieno(2,3-e)-1,2-thiazin-3-carbonsäureamid-1,1-dioxid, ein Verfahren zu ihrer Herstellung sowie ihre Verwendung**
Enolethers of 6-chloro-4-hydroxy-2-methyl-N-(2-pyridyl)-2H-thieno(2,3-e)-1,2-thiazin-3-carboxylic acid amide-1,1-dioxide, a process for their preparation and their use
Enoléthers de 6-chloro-4-hydroxy-2-méthyl-N-(2-pyridyl)-2H-thiéno(2,3-e)-1,2-thiazin-3-carboxamides-1,1-dioxyde, un procédé pour leur préparation et leur utilisation

(30) Priorität: 29.10.1987 AT 2855/87
(43) Veröffentlichungstag der Anmeldung: 03.05.1989
(73) Patentinhaber: Hafslund Nycomed Pharma Aktiengesellschaft, A-4021 Linz (AT)
(72) Erfinder: Binder, Dieter, Dr., A-1190 Wien (AT); Rovenszky, Franz, Dr., Dipl.-Ing., A-2460 Bruck a.d.Leitha (AT); Ferber, Hubert Peter, Dr., A-4021 Ansfelden (AT)
(74) Vertreter: Kunz, Ekkehard, Dr.

(56) Entgegenhaltungen:
- EP-A- 0 001 113
- EP-A- 0 147 177

## Beschreibung

Die Erfindung betrifft neue Enolether von 6-Chlor-4-hydroxy-2-methyl-N-(2-pyridyl)-2H-thieno(2,3-e)-1,2-thiazin-3-carbonsäureamid-1,1-dioxid, ein Verfahren zu ihrer Herstellung und ihre Verwendung in Medikamenten mit entzündungshemmender Wirkung.

In der US-PS 4,180,662 werden antiinflammatorisch wirksame Analgetika beschrieben. Von den in dieser US-PS beschriebenen Substanzen hat sich 6-Chlor-4-hydroxy-2-methyl-N-(2-pyridyl)-2H-thieno(2,3-e)-1,2-thiazin-3-carbonsäureamid-1,1-dioxid (Chlortenoxicam) als besonders wirksam erwiesen. Diese Verbindung kann jedoch, bedingt durch seine polare und acide Struktur in seltenen Fällen Irritationen des gastrointestinalen Traktes hervorrufen. Topische Formulierungen dieser Substanz haben den Nachteil, die Haut nur unbefriedigend zu durchdringen und übergezogene Kleidungsstücke mit ihrer intensiven gelben Farbe anzufärben.

Aus der EP-PS 0147 177 sind Oxicam-Enolether bekannt. Die in dieser EP-PS beschriebenen Enolether haben jedoch den Nachteil, daß die pharmakologische Aktivität dieser Enolether geringer ist als die Aktivität der unveretherten Oxicame.

Überraschenderweise wurde nun gefunden, daß die Enolether der vorliegenden Erfindung, die farblos sind und sich daher auch für topische Anwendungen eignen, eine größere pharmakologische Aktivität besitzen als das unveretherte Chlortenoxicam.

Gegenstand der Erfindung sind daher Verbindungen der Formel I 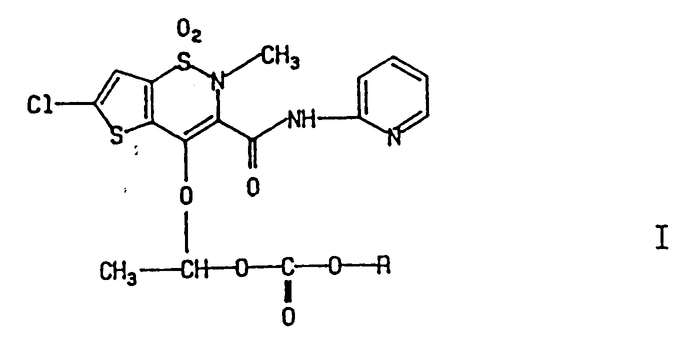
in der R (C₁ - C₆)-Alkyl, (C₅ - C₇)-Cycloalkyl oder Benzyl
bedeutet.

Der in dieser Beschreibung verwendete Ausdruck (C₁ - C₆)-Alkyl bezeichnet geradkettige oder verzweigte gesättigte Kohlenwasserstoffreste mit 1 - 6 Kohlenstoffatomen, wie z.B. Methyl, Ethyl, Isopropyl, tert. Butyl, Hexyl. Unter Halogen ist Chlor, Brom oder Jod zu verstehen.

Eine bevorzugte Einzelverbindung ist:
6-Chlor-4-(1-(ethoxycarbonyloxy)ethoxy)-2-methyl-N-(2-pyridyl)-2H-thieno(2,3-e)-1,2-thiazin-3-carbonsäureamid-1,1-dioxid.

Die Verbindungen der Formel I werden dadurch hergestellt, daß man ein Salz des Chlortenoxicams der Formel II 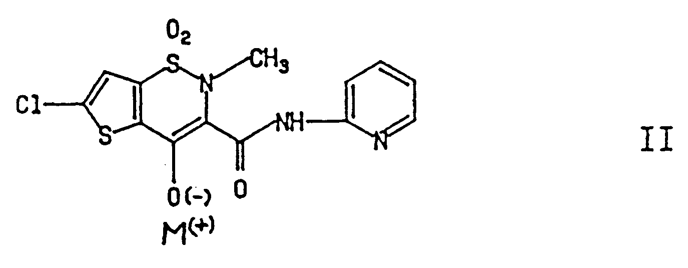
in der M⁺ ein Alkali- oder
Erdalkalikation oder Tetraalkylammonium bedeutet, mit einer Verbindung der Formel III 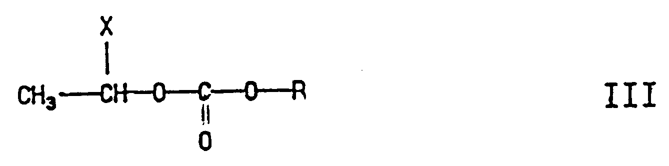
in der R die obige Bedeutung hat und X Halogen bedeutet, in einem reaktionsinerten polaren aprotischen Lösungsmittel umsetzt.

Die erforderlichen Salze des Chlortenoxicams können isoliert eingesetzt werden; besser erzeugt man sie in situ durch Zugabe von mindestens einem Äquivalent einer starken Base wie z.B. Alkalihydride oder Alkalicarbonate zur Reaktionslösung.

Die Umsetzung der Verbindung der Formel II mit einer Verbindung der Formel III findet in einem reaktionsinerten, polaren, aprotischen, wasserfreien Lösugsmittel wie z.B. DMF, DMSO, Aceton, 2-Butanon, etc. statt. Die Reaktionstemperatur ist nicht kritisch und liegt zwischen Raumtemperatur und der Siedetemperatur des jeweils verwendeten Lösungsmittels. Die Reaktionsdauer ist von der Reaktionstemperatur und der Abgangsgruppe X abhängig; im allgemeinen liegt sie zwischen 2 und 30 Stunden. Die Reaktion läßt sich durch Zusatz von Natriumjodid (Finckelstein-Reaktion) beschleunigen, wobei NaJ in einem bis zu dreifachen Überschuß, bezogen auf das Alkylierungsmittel, eingesetzt wird.

Bevorzugte Reaktionsbedingungen sind der Umsatz des Chlortenoxicams mit Verbindungen der Formel III in Aceton als Lösungsmittel und Natrium- oder Kaliumcarbonat als Base in einem 3 - 4-fachen Überschuß bei Rückflußtemperatur und NaJ als Reaktionsbeschleuniger, wobei 1,5 - 2 Mol NaJ pro Mol Alkylierungsmittel eingesetzt werden.

Chlortenoxicam kann gemäß US-PS 4,180,662 hergestellt werden. Die Verbindungen der Formel III sind entweder im Handel erhältlich oder können gemäß H. Müller, J. Liebigs Ann. Chem. 258, 50 (1890) oder nach EP-PS 0147 177 hergestellt werden.

Die neuen Verbindungen der Formel I zeigen in in vitro Modellen eine hervorragende entzündungshemmende Aktivität.

Aufgrund dieser pharmakologischen Eigenschaft können die neuen Verbindungen allein oder in Mischung mit anderen Wirksubstanzen in Form üblicher galenischer Zubereitungen zur Entzündungshemmung und Schmerzbekämpfung bei Erkrankungen wie Rheuma verwendet werden.

Die entzündungshemmende Eigenschaft kann mittels allgemein bekannter Standardmethoden wie beispielsweise dem Carrageenan-induzierten Rattenpfoten-Schwellungstest bestimmt werden. In diesem Test (Beispiel 2), in dem Chlortenoxicam, ein Enolether des Chlortenoxicams, nämlich 6-Chlor-4-(1-(ethoxycarbonyloxy)ethoxy)-2-methyl-N-(2-pyridyl)-2H-thieno(2,3-e)-1,2-thiazin-3-carbonsäureamid-1,1-dioxid, Piroxicam (2-Methyl-N-(2-pyridyl)-4-hydroxy-2H-1,2-benzothiazin-3-carbonsäureamid-1,1-dioxid) und ein Enolether des Piroxicams, nämlich 4-((1-Ethoxycarbonyloxy)ethoxy)-2-methyl-N-(2-pyridyl)-2H-1,2-benzothiazin-3-carbonsäureamid-1,1-dioxid in ihrer entzündungshemmenden Aktivität verglichen wurden, zeigte sich, daß unter den gegebenen Versuchsbedingungen nur mit dem Enolether des Chlortenoxicams eine 80 %ige Entzündungshemmung zu erreichen war. Aus den Werten für die 50 %ige Entzündungshemmung läßt sich ableiten, daß der Enolether des Chlortenoxicams fast doppelt so wirksam ist als Chlortenoxicam, während der Enolether des Piroxicams wesentlich schwächer wirksam ist wie Piroxicam. Es ergibt sich daher folgende Reihung abnehmender entzündungshemmender Potenz:
Chlortenoxicam-Enolether/Chlortenoxicam/Piroxicam/Piroxicam-Enolether
Die Verbindungen der Formel I sind zur Verwendung an Säugetieren, insbesondere am Menschen bestimmt und können auf übliche Weise, wie beispielsweise oral oder parenteral, verabreicht werden. Vorzugsweise werden sie oral oder topisch verabreicht, wobei die Tagesdosis bei oraler Gabe ca. 0,5 bis 100 mg beträgt, vorzugsweise 1,0 bis 10 mg. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben. Bei topischer Verabreichung liegt die Konzentration der Verbindung der Formel I zwischen 0,01 und 3 %.
Die Verbindungen der Formel I können allein oder in Verbindung mit anderen pharmazeutisch aktiven Substanzen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, wie z.B. pharmazeutisch unbedenklichen Lösungsmitteln, Gelatine, Gummi arabicum, Milchzucker, Stärke , Magnesiumstearat, Talk, pflanzlichen Ölen, Polyalkylenglykol, Vaseline und dergleichen vor.

Die pharmazeutischen Präparate können in fester Form, beispielsweise als Tabletten, Dragees, Suppositorien, Kapseln und dergleichen, in halbfester Form, beispielsweise als Salben oder Gel oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs-oder Emulgiermittel, Salze zur Veränderung des osmotischen Druckes und dergleichen.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

### Beispiel 1:

### 6-Chlor-4-(1-(ethoxycarbonyloxy)ethoxy)-2-methyl-N-(2-pyridyl)-2H-thieno(2,3-e)-1,2-thiazin-3-carbonsäureamid-1,1-dioxid

10 g (26,9 mmol) 6-Chlor-4-hydroxy-2-methyl-N-(2-pyridyl)-2H-thieno-(2,3-e)-1,2-thiazin-3-carbonsäureamid-1,1-dioxid, 9,29 g (99,5 mmol) Kaliumcarbonat und 15,1 g (99,5 mmol) 1-Chlorethylethylcarbonat werden in 150 ml Aceton 20 Stunden unter Rückfluß erhitzt. Anschließend werden 24,5 g (163,3 mmol) Natriumjodid zugegeben und weitere 5 Stunden unter Rückfluß erhitzt. Danach wird das ausgefallene Natriumchlorid abgesaugt, das Filtrat eingedampft und zwischer 100 ml Methylenchlorid und 100 ml gesättigter Natriumbicarbonatlösung verteilt. Die Phasen werden getrennt und die organische Phase mit 100 ml Wasser und 20 ml 3 %iger Natriumbisulfitlösung gewaschen. Es wird über Natriumsulfat getrocknet, filtriert und eingedampft. Das erhaltene ölige Rohprodukt (17,5 g) wird über Kieselgel filtriert (100 g Kieselgel 60, Korngröße 0,04 - 0,063 mm, Eluens: Methylenchlorid : Ethylacetat = 9 : 1). Es werden 10,1 g hellorange Kristalle erhalten. Diese werden in 17 ml Dioxan in der Siedehitze gelöst, die Lösung mit 0,6 g Aktivkohle versetzt und anschließend heiß filtriert. Es wird abgekühlt und mit 40 ml Diethylether versetzt. Die ausgefallenen farblosen Kristalle werden abgesaugt, mit Ether gewaschen und bei 50 °C /1 mbar getrocknet.
Ausbeute: 6,3 g farblose Kristalle (48 % d.Th.)
Schm.: 148 °C (Zersetzung)
¹H-NMR (CDCl₃):
delta (ppm): 8,9 (s breit; 1H; -NH-); 8,3 (m; 2H; Py-H); 7,8 (m; 1H; Py-H); 7,2 (s; 1H; Th-H); 7,1 (m; 1H; Py-H); 6,5 (q; 1H; 0-CH-0); 4,1 (q; 2H; - CH₂-); 3,2 (s; 3H; N-CH₃); 1,7 (d; 3H; -CH-CH₃); 1,2 (t; 3H; -CH₂-CH₃).
¹³C-NMR (CDCl₃):
delta (ppm): 157,9; 153,1; 150,5; 147,9; 142,1; 137,9; 135,9; 135,5; 134,9; 126,6; 121,1; 120,1; 113,9; 100,1; 64,4; 36,7; 19,9; 13,7.

### Beispiel 2:

### Carrageenan-induzierter Rattenpfoten-Schwellungstest

Die entzürdungshemmende Wirkung der Testsubstanzen wurde durch ihre Hemmwirkung auf die Carrageenan-induzierte Rattenpfotenschwellung geprüft.

Als Testsubstanzen dienten:
Chlortenoxicam ( 6-Chlor-4-hydroxy-2-methyl-N-(2-pyridyl)-2H-thieno(2,3-e)-1,2-thiazin-3-carbonsäureamid-1,1-dioxid )
Chlortenoxicam-Enolether ( 6-Chlor-4-(1-(ethoxycarbonyloxy)ethoxy)-2-methyl-N-(2-pyridyl)-2H-thieno(2,3-e)-1,2-thiazin-3-carbonsäureamid-1,1-dioxid )
Piroxicam ( 2-Methyl-N-(2-pyridyl)-4-hydroxy-2H-1,2-benzothiazin-3-carbonsäureamid-1,1-dioxid )
Piroxicam-Enolether ( 4-(1-(Ethoxycarbonyloxy)ethoxy)-2-methyl-N-(2-pyridyl)-2H-1,2-benzothiazin-3-carbonsäureamid-1,1-dioxid )
Vor Versuchsbeginn wurde das Volumen der rechten Rattenhinterpfoten plethysmometrisch bestimmt und in ml Wasserverdrängung festgehalten.
Die Testsubstanzen wurden als Suspension in 0,5 %iger Carboxymethylzellulose oral mittels Schlundsonde appliziert. Die Dosierung betrug 0,3 / 1,0 / 3,0 und 10 mg/kg Körpergewicht. Pro Substanz und Dosis bzw. Kontrolle wurden 8 Tiere getestet. Nach einer Stunde wurde die Entzündung durch eine Injektion von 0,05 ml einer 2 %igen Lösung von Lambda Carrageenan in 0,9 % NaCl in die rechten Hinterpfoten der Versuchstiere ausgelöst. 3 und 4 Stunden nach der Entzündungsauslösung wurde eine weitere plethysmometrische Volumsbestimmung der rechten Rattenhinterpfoten vorgenommen. Die Entzündungshemmung wird in % angegeben. Aus diesen Werten wird die 80 % und die 50 % IHD (Inhibition Dosis) ausgerechnet. (Die 80 % IHD gibt jene Dosis in mg /kg Körpergewicht an, die imstande ist, eine Entzündung zu 80 % zu inhibieren).

| 80 % IHD-Werte: | | | |
|---|---|---|---|
| Substanz | 3 h nach Carrageenan (mg/kg) | 4 h nach Carrageenan (mg/kg) | geometrischer Mittelwert (mg/kg) |
| Chlortenoxicam-Enolether | 3,01 | 3,03 | 3,02 |
| Chlortenocicam | n.e | n.e | n.e |
| Piroxicam | n.e | n.e | n.e |
| Piroxicam-Enolether | n.e | n.e | n.e |
| n.e: nicht erreichbar | | | |

| 50 % IHD-Werte: | | | |
|---|---|---|---|
| Substanz | 3 h nach Carrageenan (mg/kg) | 4 h nach Carrageenan (mg/kg) | geometrischer Mittelwert (mg/kg) |
| Chlortenoxicam-Enolether | 0,23 | 0,36 | 0,29 |
| Chlortenoxicam | 0,35 | 0,60 | 0,46 |
| Piroxicam | 3,88 | 5,06 | 4,43 |
| Piroxicam-Enolether | g.a.10 | g.a.10 | g.a.10 |
| g.a.: größer als | | | |

### Beispiel 3:

### Herstellung einer Chlortenoxicam-Enolether-Gel-Charge

In einer FRYMA-Prozeßanlage werden 8 g 6-Chlor-4-(1-(ethoxycarbonyloxy)ethoxy)-2-methyl-N-(2-pyridyl)-2H-thieno(2,3-e)-1,2-thiazin-3-carbonsäureamid-1,1-dioxid in 4 717 g Äthanol und 2 082 g Wasser gelöst. In diese Lösung werden 167 g Carbopol unter Rühren portionsweise eingetragen. Nach Zugabe von 139 g Luvitol EHO wird der Ansatz mit einer aus 83 g Diisopropylamin, 833 g Äthanol und 833 g Wasser bereiteten Lösung neutralisiert und anschließend mit einer aus 28 g Diisopropylamin, 555 g Äthanol und 555 g Wasser bestehenden Lösung auf einen pH-Wert von 7,5 eingestellt. Das Gel wird in Tuben abgefüllt.

## Patentansprüche (Patentansprüche für folgende(n) Vertragsstaat(en): AT, BE, CH, DE, FR, GB, IT, LI, LU, NL, SE)

1. Verbindungen der Formel I 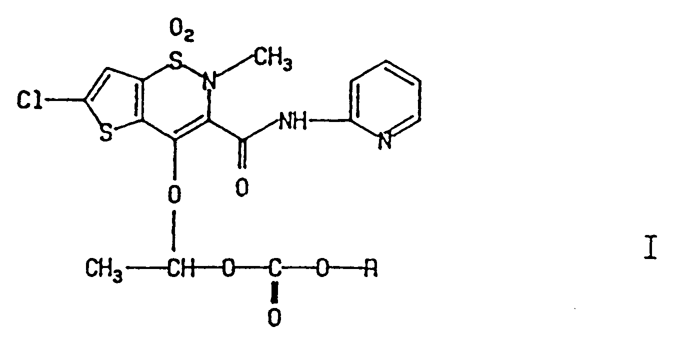 in der R (C₁ - C₆)-Alkyl, (C₅ - C₇)-Cycloalkyl oder Benzyl bedeutet.

2. 6-Chlor-4-(1-(ethoxycarbonyloxy)ethoxy)-2-methyl-N-(2-pyridyl)-2H-thieno-(2,3-e)-1,2-thiazin-3-carbonsäureamid-1,1-dioxid, gemäß Formel (I) nach Anspruch 1.

3. Verfahren zur Herstellung von Verbindungen der Formel (I), gemäß Anspruch 1, dadurch gekennzeichnet, daß man ein Salz des Chlortenoxicams der Formel II 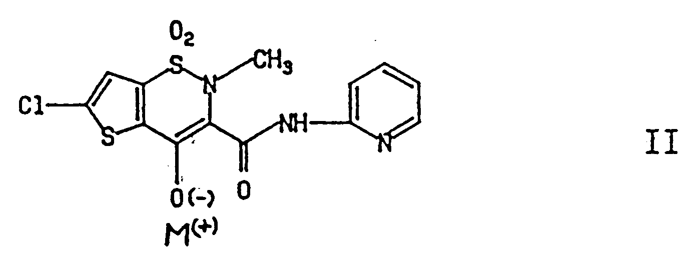 in der M⁺ ein Alkali-oder Erdalkalikation oder Tetraalkylammonium bedeutet, mit einer Verbindung der Formel III 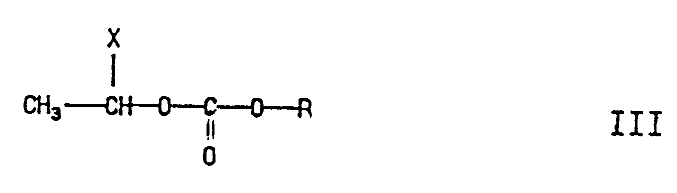 in der R die obige Bedeutung hat und X Halogen bedeutet,
in einem reaktionsinerten polaren aprotischen Lösungsmittel umsetzt.

4. Pharmazeutische Präparate, enthaltend Verbindungen der Formel I in Kombination mit üblichen galenischen Hilfs- und/oder Trägerstoffen oder Verdünnungsmitteln.

5. Pharmazeutische Präparate, enthaltend Verbindungen der Formel I gemäß Anspruch 1, in Kombination mit anderen therapeutisch wertvollen Wirkstoffen sowie üblichen galenischen Hilfs- und/oder Trägerstoffen oder Verdünnungsmitteln.

6. Verbindungen der Formel I, gemäß Anspruch 1, zur Verwendung als Wirk stoffe für Arzneimittel zur Behandlung von entzündlichen Erkrankungen.

## Patentansprüche (Patentansprüche für folgende(n) Vertragsstaat(en): ES, GR)

1. Verfahren zur Herstellung von Verbindungen der Formel I 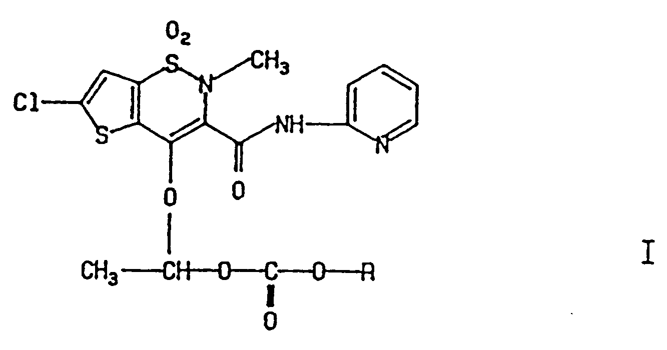 in der R (C₁ - C₆)-Alkyl, (C₅ - C₇)-Cycloalkyl oder Benzyl bedeutet, dadurch gekennzeichnet, daß man ein Salz des Chlortenoxicams der Formel II 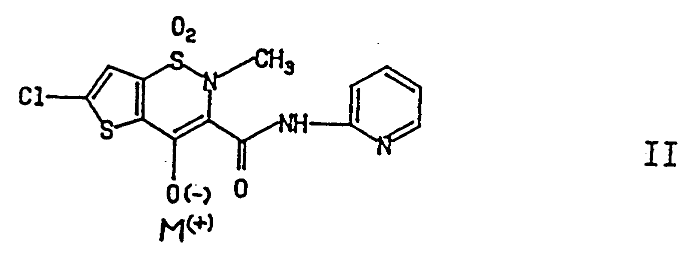 in der M⁺ ein Alkali-oder Erdalkalikation
oder Tetraalkylammonium bedeutet, mit einer Verbindung der Formel III 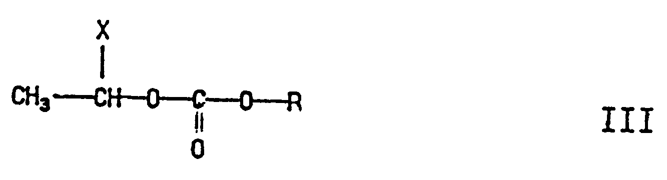 in der R die obige Bedeutung hat und X Halogen bedeutet, in einem reaktionsinerten polaren aprotischen Lösungsmittel umsetzt.

2. Verfahren nach Anspruch 1, dadurch gekennzeichnet, daß man das Salz des Chlortenoxicams in situ durch Zugabe von mindestens einem Äquivalent einer starken Base, bevorzugt in einem 3- bis 4-fachen Überschß einer starken Base, in der Reaktionslösung herstellt.

3. Verfahren nach einem der Ansprüche 1 oder 2, dadurch gekennzeichnet, daß die starke Base ein Alkalicarbonat ist.

4. Verfahren nach einem der Ansprüche 1 bis 3, dadurch gekennzeichnet, daß die starke Base ein Kaliumcarbonat ist.

5. Verfahren nach einem der Ansprüche 1 bis 4, dadurch gekennzeichnet, daß das reaktionsinerte polare Lösungsmittel Aceton ist.

6. Verfahren nach einem der Ansprüche 1 bis 5, dadurch gekennzeichnet, daß man die Umsetzung des Salzes des Chlortenoxicams mit einer Verbindung der Formel II bei der Rückflußtemperatur des Lösungsmittels durchführt.

7. Verfahren nach einem der Ansprüche 1 bis 6, dadurch gekennzeichnet, daß man zur Reaktionsbeschleunigung Natriumjodid in einem bis zu 3-fachem Überschuß, bezogen auf das Alkylierungsmittel, einsetzt.

8. Verfahren nach einem der Ansprüche 1 bis 7, dadurch gekennzeichnet, daß man 1,5 - 2,0 Mol Natriumjodid pro Mol Alkylierungsmittel einsetzt.

## Claims (Claims for the following Contracting State(s): AT, BE, CH, DE, FR, GB, IT, LI, LU, NL, SE)

1. Compounds of the formula I 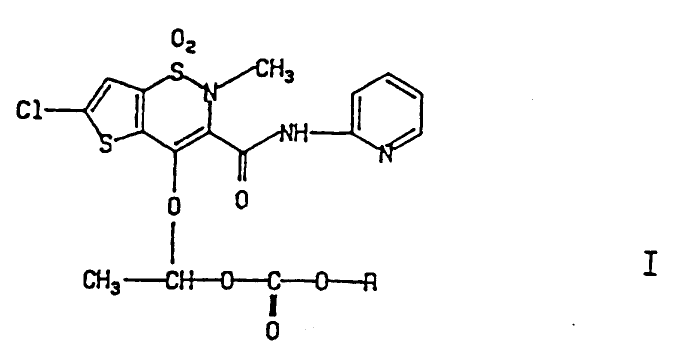 in which R denotes (C₁-C₆)-alkyl, (C₅-C₇)-cycloalkyl or benzyl.

2. 6-Chloro-4-(1-(ethoxycarbonyloxy)ethoxy)-2-methyl-N-(2-pyridyl)-2H-thieno-(2,3-e)-1,2-thiazine-3-carboxamide 1,1-dioxide in accordance with formula (I) according to Claim 1.

3. Process for the preparation of compounds of the formula (I), according to Claim 1 characterized in that a salt of chlortenoxicam of the formula II 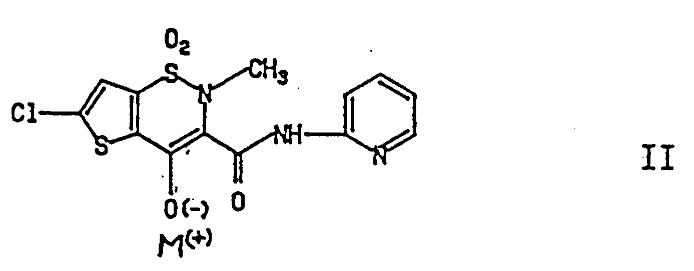 in which M⁺ denotes an alkali metal cation or alkaline earth metal cation or tetraalkylammonium, is reacted with a compound of the formula III 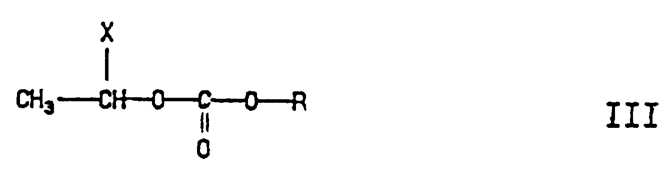 in which R has the above meaning and X denotes halogen, in a polar aprotic solvent which is inert towards the reaction.

4. Pharmaceutical preparations containing compounds of the formula I in combination with customary pharmaceutical auxiliaries and/or excipients or dilutents.

5. Pharmaceutical preparations containing compounds of the formula I according to Claim 1 in combination with other therapeutically useful active compounds and customary pharmaceutical auxiliaries and/or excipients or diluents.

6. Compounds of the formula I according to Claim 1 for use as active compounds for medicaments for the treatment of inflammatory diseases.

## Claims (Claims for the following Contracting State(s): ES, GR)

1. Process for the preparation of compounds of the formula I 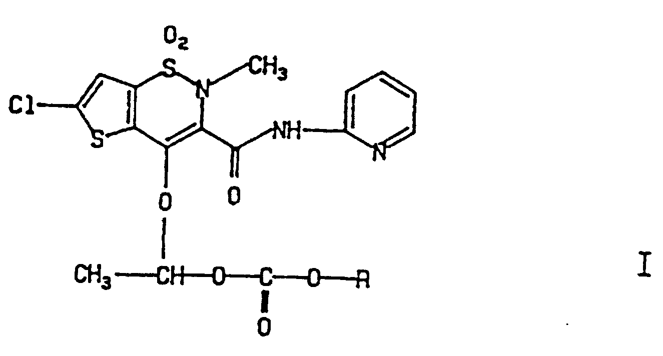 in which R denotes (C₁-C₆)-alkyl, (C₅-C₇)-cycloalkyl or benzyl, characterized in that a salt of chlortenoxicam of the formula II 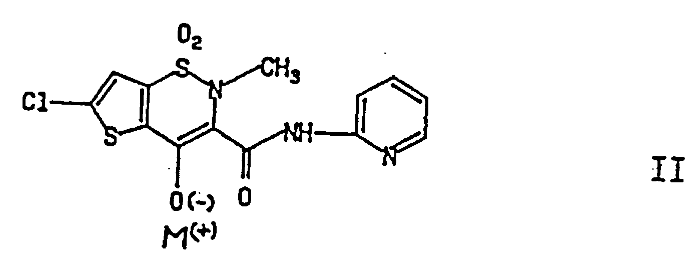 in which M⁺ denotes an alkali metal cation or alkaline earth metal cation or tetraalkylammonium, is reacted with a compound of the formula III 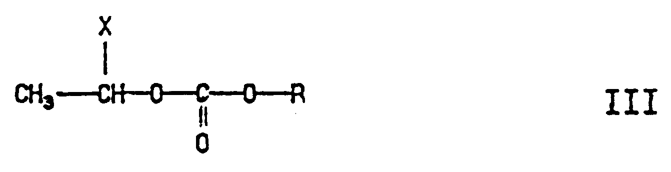 in which R has the above meaning and X denotes halogen, in a polar aprotic solvent which is inert towards the reaction.

2. Process according to Claim 1, characterized in that the salt of chlortenoxicam is prepared in situ by addition of at least one equivalent of a strong base to the reaction solution, preferably in a 3- to 4-fold excess of the strong base.

3. Process according to one of Claims 1 or 2, characterized in that the strong base is an alkali metal carbonate.

4. Process according to one of Claims 1 to 3, characterized in that the strong base is a potassium carbonate.

5. Process according to one of Claims 1 to 4, characterized in that the polar solvent which is inert towards the reaction is acetone.

6. Process according to one of Claims 1 to 5, characterized in that the reaction of the salt of chlortenoxicam with a compound of the formula II is carried out at the reflux temperature of the solvent.

7. Process according to one of Claims 1 to 6, characterized in that sodium iodide is employed in an excess of up to 3-fold, relative to the alkylating agents, to accelerate the reaction.

8. Process according to one of Claims 1 to 7, characterized in that 1.5 - 2.0 mol of sodium iodide are employed per mol of alkylating agent.

## Revendications (Revendications pour l'(les) Etat(s) contractant(s) suivant(s): AT, BE, CH, DE, FR, GB, IT, LI, LU, NL, SE)

1. Combinaisons de la formule I 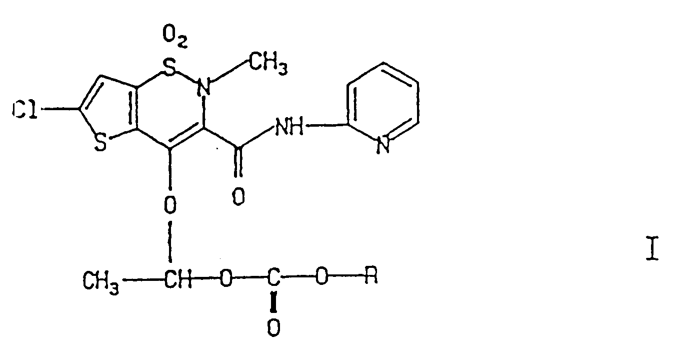 dans laquelle R signifie (C₁ - C₆)-alkyle, (C₅-C₇)-cycloalkyle ou benzyle.

2. 6-chloro-4-(1-(éthoxycarbonyloxy)éthoxy)-2-méthyl-N-(2-pyridyl)-2H-thiéno(2,3-e)-1,2-thiazine-3-amide d'acide carboxylique-1,1-dioxyde, selon la formule (I) selon revendication 1.

3. Procédé de fabrication de combinaisons de la formule I selon revendication 1,
caractérisé par le fait que l'on transpose un sel du chlorténoxicam de la formule II 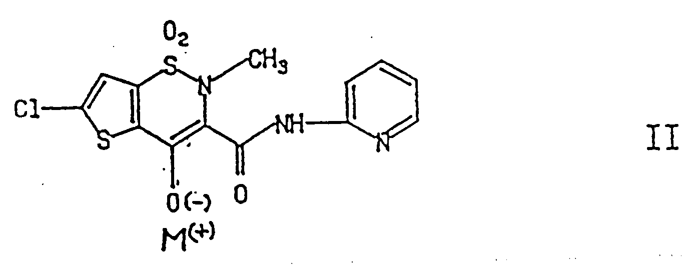 dans laquelle M+ signifie un cation alcalin, un cation alcalino-terreux ou un ammonium tétraalkyle, avec une combinaison de la formule III 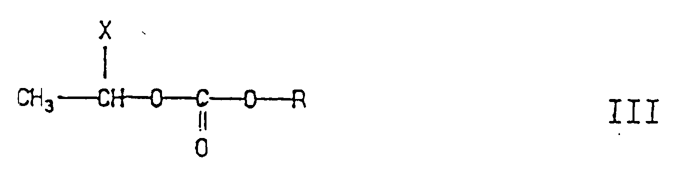 dans laquelle R a la signification ci-dessus mentionnée et X signifie un halogène, dans un solvant polaire, aprotique, de comportement inerte à la réaction.

4. Préparations pharmaceutiques contenant des combinaisons de la formule I selon revendication 1 combinées à des substances galéniques auxiliaires et/ou support habituelles ou encore à des diluants.

5. Préparations pharmaceutiques contenant des combinaisons de la formule I, selon revendication 1 en combinaison avec d'autres substances thérapeutiques efficaces ainsi que des substances galéniques auxiliaires et/ou support habituelles ou encore à des diluants.

6. Combinaisons de la formule I, selon revendication 1, pour l'utilisation comme substances actives de médicaments utilisés dans le traitement des inflammations.

## Revendications (Revendications pour l'(les) Etat(s) contractant(s) suivant(s): ES, GR)

1. Procédé de fabrication de combinaisons de la formule I 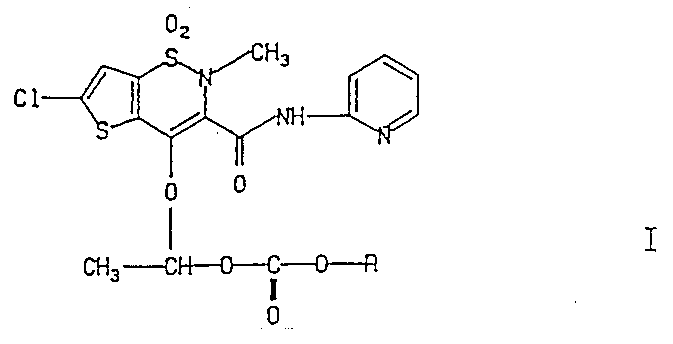 dans laquelle R signifie (C₁ - C₆)-alkyle, (C₅-C₇)-cycloalkyle ou benzyle, caractérisé par le fait que l'on transpose un sel du chlorténoxicam de la formule II 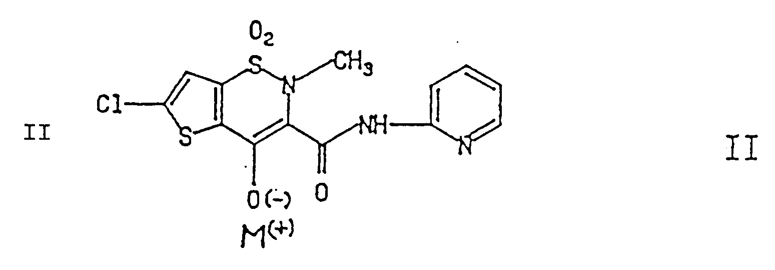 dans laquelle M+ signifie un cation alcalin, un cation alcalino-terreux ou un ammonium tétraalkyle, avec une combinaison de la formule III 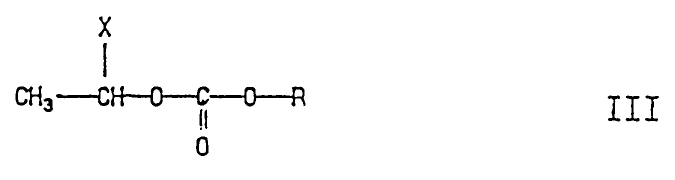 dans laquelle R a la signification ci-dessus mentionnée et X signifie un halogène, dans un solvant polaire, aprotique, de comportement inerte à la réaction.

2. Procédé selon revendication 1, caractérisé par le fait que l'on produit le sel du chlorténoxicam in situ en ajoutant à la solution réactive au moins un équivalent d'une forte base, de préférence dans un triple ou quadruple surplus d'une forte base.

3. Procédé selon l'une des revendications 1 ou 2, caractérisé par le fait que la forte base est constituée par un alcalicarbonate.

4. Procédé selon l'une des revendications 1 à 3, caractérisé par le fait que la forte base est constituée par un carbonate de potassium.

5. Procédé selon l'une des revendications 1 à 4, caractérisé par le fait que le solvant polaire inerte à la réaction est de l'acétone.

6. Procédé selon l'une des revendications 1 à 5, caractérisé par le fait que le sel du chlorténoxicam est transposé avec une combinaison de la formule II à température de reflux du solvant.

7. Procédé selon l'une des revendications 1 à 6, caractérisé par le fait que l'on utilise pour l'accélération de la réaction du iodure de sodium à un surplus pouvant aller jusqu'au triple par rapport à l'agent d'alkylation.

8. Procédé selon l'une des revendications 1 à 7, caractérisé par le fait que l'on utilise 1,5 - 2,0 mol. de iodure de sodium par mol. d'agent d'alkylation.
